# EUROPEAN PATENT APPLICATION

(11) **EP 1 815 829 A1**
(43) Date of publication of application: **08.08.2007**
(21) Application number: 07250243.8
(22) Date of filing: 22.01.2007
(51) Int. Cl.: A61F 5/453, A61F 5/455

(54) **Fluid impermeable cover for male and female genitalia**

(30) Priority: 28.01.2006 US 307250
(71) Applicant: Ferriss, Nadine, Ottawa, Ontario K1L 8L3 (CA); Boucher, Tammy, Ottawa, Ontario K1L 8L3 (CA)
(72) Inventor: Ferriss, Nadine, Ottawa, Ontario K1L 8L3 (CA); Boucher, Tammy, Ottawa, Ontario K1L 8L3 (CA)
(74) Representative: Hall, Matthew Benjamin

(57) **Abstract**

In the procedure of waxing or laser treatment for body hair removal, particularly body hair removal near male and female genitalia, a device in the form of a pouch (11) for males, and an inverted oval-shaped cup (2) for females, is used to cover the genitalia. The pouch or cup, along with a wide thong-type rear band (6) over the rectum prevent transmission of body fluids during the procedure.

## Description

This invention relates to covers for the male and female genitalia to prevent bodily fluid transmission during a waxing of body hair procedure. The covers also add to the personal security and comfort of the wearer and the person carrying out the waxing operation. The covers are also used during laser hair removal.

Body hair removal, particularly pubic hair in the genital areas of both males and females has become more popular in recent decades. The most popular procedure is the use of the application of warm or hot wax which is later peeled away, removing the hair follicles. Epilation is the common name used by aestheticians. Another procedure which offers permanent pubic hair removal is that using lasers.

More recently, the removal of all hair from front to back of the genitalia has been undertaken and has the coined name of the "Brazilian wax". In order to carry out this procedure the inventors have found that it is necessary only to cover the inner labia of a female vulva to prevent fluid transmission and allow all pubic hair to be removed. However, many men and women undertaking the waxing hair-removal procedure prefer to have only a portion of the pubic hair removed.

The Brazilian wax was developed in New York in about 1987 by a number of Brazilian-born sisters and is now becoming popular worldwide. Nevertheless, there now appear to be many variations to the Brazilian wax in that decorative patterns can be applied to the pubic hair during such removal.

The waxing technique is generally carried on not at a doctor's office or hospital setting, but rather a salon operated by licensed aestheticians. The salon may or may not be associated with a hair salon. Such aestheticians generally wear face masks and other protective garments, such as plastic gloves, when dealing at close proximity with male and female genitalia.

Because of the increase in the HIV virus and other sexually-transmitted diseases which are easily transmitted in bodily fluids emitted from the male genitalia, female vulva, and anal areas of both males and females, there is some concern about the waxing procedure in the profession.

Presently the male or female is either completely nude or wears some type of thong or undergarment during the waxing procedure. During laser hair removal gauze is placed between the buttocks and over the inner labia of a female. This has proven to be not only cumbersome for the wearer, but also poses problems for the operator or aesthetician performing the waxing technique, a laser hair removal.

The present inventors have identified that it would be desirable to provide a covering device which prevents transmission of bodily fluids from the rectum and the male and female genitalia during the hair removal process. It would also be advantageous to provide a cover for the male and female genitalia and rectum to provide more private, personal security for the wearer, and to permit the operator to feel more comfortable in a rather personal procedure.

Therefore this invention seeks to provide a device for preventing transmission of human body fluids emitted from male and female genitalia and rectum during a pubic hair removal procedure, comprising: a fluid impermeable cover; said cover adapted in operation to fit securely over, at least inner labia, of a female vulva and penis of a male; said device further comprising a wide fluid impermeable band secured to a lower end of said cover and adapted in operation to cover a rectum of a human body; said cover and said wide fluid-impermeable band being secured in place by a plurality of adjustable, stretchable straps; said straps including at least one adjustable waistband, at least one adjustable, stretchable front strap, and an adjustable, stretchable rear strap; said front strap being attached at its lower end to an upper end of said cover, and at its upper end stretchably secured to said adjustable waistband; said rear strap being attached at its lower end to an upper end of said wide fluid impermeable strap, and at its upper end to said adjustable waistband.

The invention includes many embodiments. For example some women prefer to have both the labia minora and the labia majora, i.e. the complete vulva, entirely covered by the device of the present invention during waxing or laser hair removal. As such this is encompassed by the preferred embodiments of the present invention.

It is also preferred to include on an inner surface or outer surface of the cover or the wide fluid impermeable band a scented strip to reduce any odor emissions from the vagina or the rectum respectively.

It is also contemplated in the preferred embodiments to provide an outside surface of the cover which is non-stick or wax repellant. Such a surface may be made of Teflon^{(™)} or some other suitable material.

It is also preferred to provide a cover constructed of cellulose or other material which is suitable for gentle contact with the female genitalia.

It is also preferred to have the straps being detachably connected to the cover and pouch; the cover and pouch being disposable after the waxing operation and the straps being washable and reusable.

The basic invention has numerous variations including various means, such as buckles or stretchable fabric, to secure in place the cover and the rear wide fluid impermeable band. The female embodiment of the invention also includes various covers in decorative shapes attached to the upper portion of the cover and connected to the waistband for contour sculpturing of the pubic hair. Covers of any or all decorative shapes are possible.

Certain preferred embodiments will now be described in greater detail, by way of example only and with reference to the accompanying figures, in which:
Figure 1 is a front view of the female body with a preferred cover of the present invention covering the inner labia of a female body;
Figure 2 is a front perspective view of a female body wearing a preferred embodiment of the present invention;
Figure 3 is a rear view of a female wearing the device of Figure 2;
Figure 4a is an enlarged view of the pelvic area of the female wearing the device of Figure 2;
Figure 4b is an enlarged view of the pelvic area of the female wearing the device of Figure 2 demonstrating the adjustability of the straps;
Figure 5a is an enlarged view of the buttocks area of a female wearing a preferred device;
Figure 5b is a rear view of the buttocks area of a female wearing a preferred device in an adjusted position;
Figure 6a is a front view of an additional embodiment of the present invention having a "zigzag" pattern shaped piece of material attached above the cover of the present invention;
Figure 6b is a heart shaped configuration embodiment attached above the cover of the present invention;
Figure 6c is a decorative configuration attached above the cover of the present invention;
Figure 6d is another embodiment of a pubic hair sculpting configuration attached above the cover in the frontal area of the female pelvic region;
Figure 7 is a front perspective view of a portion of a man's body wearing a preferred embodiment of the present invention; and
Figure 8 is a rear perspective view of a man's body wearing the preferred embodiment of Figure 7.

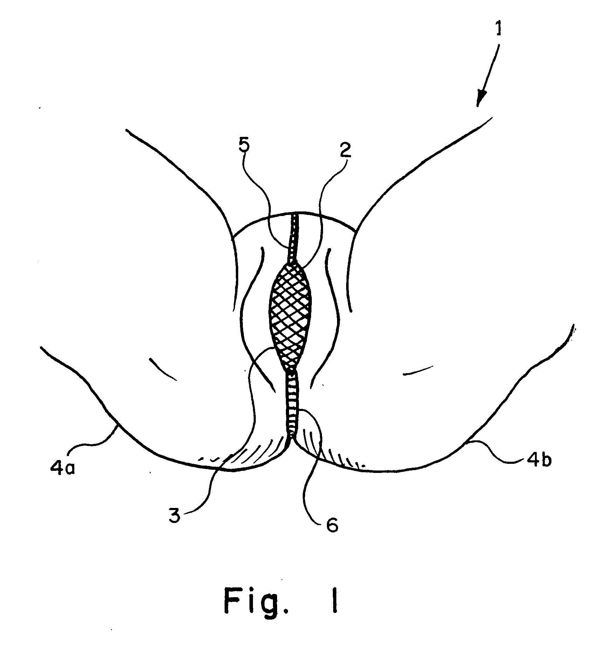

Figure 1 is a schematic view of a female 1 in an uncompromising position with a cover 2 of the present invention attached over the inner labia area 3 of the vulva. The schematic drawing in Figure 1 shows a pair of legs 4a and 4b of a female human. A thin elastic tightening strap 5 is attached to the upper end of the protective cover or cup 2 and a wide, thick strap 6 is attached to the bottom or lower end of the cup 2 and is positioned over the peritonea and between the buttocks.

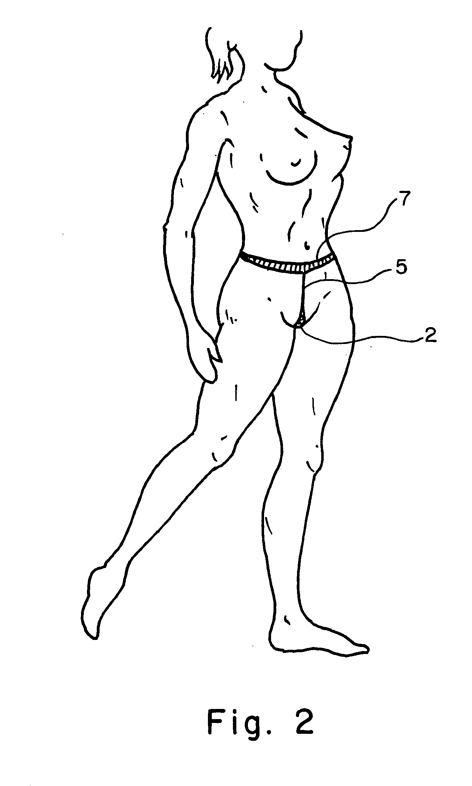

Figure 2 is a perspective view of a female in an upright position. The device of the present invention is shown generally as having a cup or cover 2 attached to a thin elastic tightening strap 5, attached to an adjustable waist band strap 7.

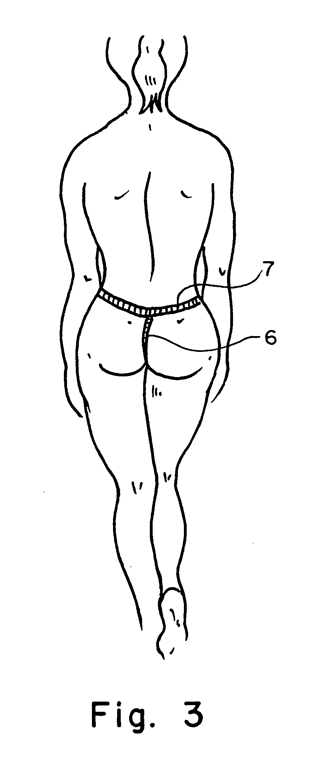

Figure 3 is a rear view of the same female figure as seen in Figure 2 showing the wide thick strap 6 being attached at its upper end to the waistband 7.

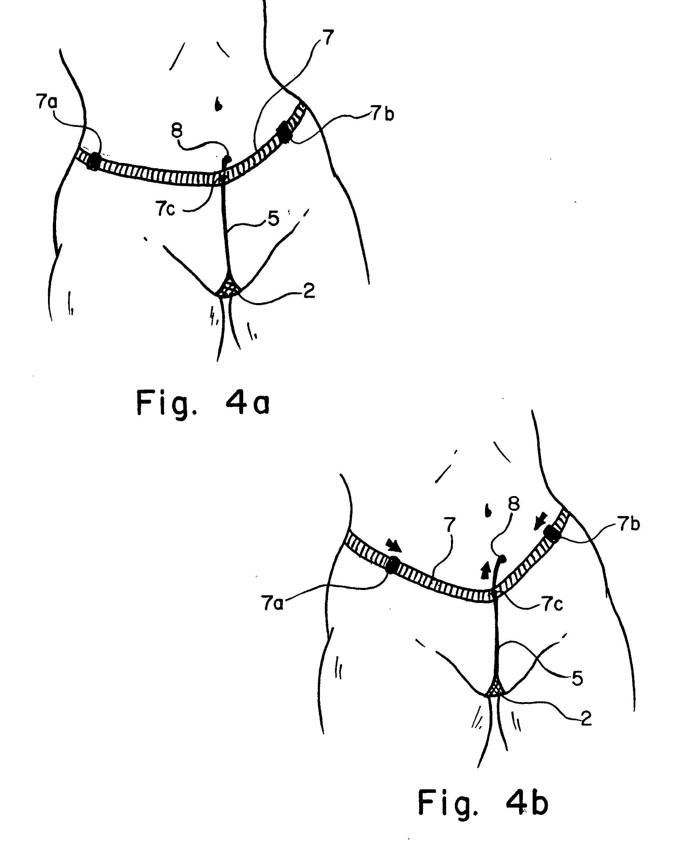

Figure 4a is a front view of a female pelvic area wherein the female is wearing the device of the present invention. Figure 4a is somewhat enlarged over the previous drawings. Waistband 7 contains adjustable buckles 7a and 7b which are capable of adjusting the waistband. In addition, the waistband 7 contains an aperture in its midportion, namely aperture 7c which is adapted to receive elastic, stretchable strap 5 which is thereafter knotted 8 to secure contact of the cover 2 over the inner labia of the vulva of the female.

In Figure 4b a similar view is shown with the elastic front strap 5 having been pulled through the aperture 7c and tightened into a knot 8. As well, buckles 7a and 7b have been tightened to increase contact of cup/cover 2 so that fluid transmission is prevented.

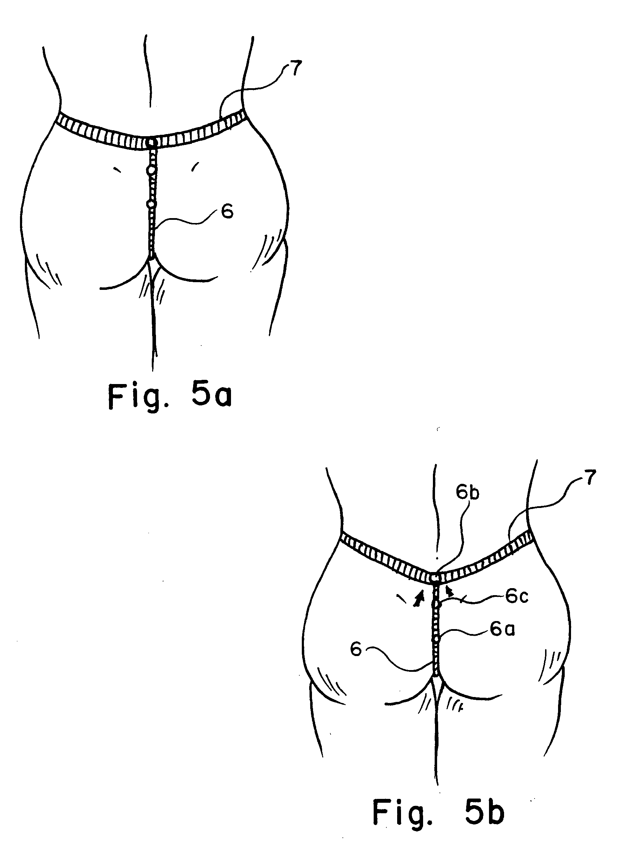

Figure 5a is a rear enlarged view of the buttocks of a female showing the wide, protective rear strap 6 connected to an adjustable rear strap 6c which is connected to the waistband 7.

Figure 5b is a similar to Figure 5a however pressure has been applied to the wide rectum covering strap 6 by means of buckles, clasps or Velcro 6a and 6b to tension the strap between the buttocks of the wearer.

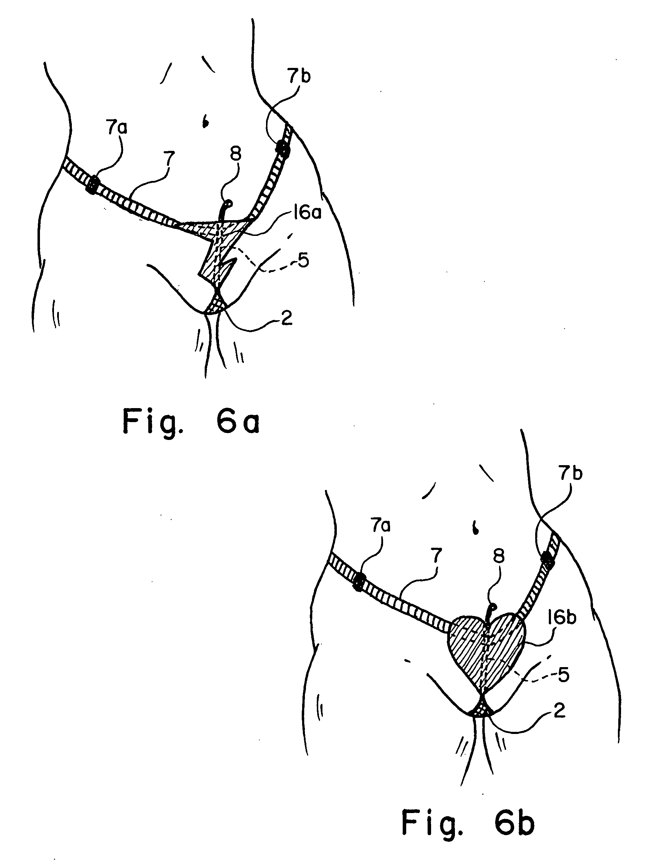

Figures 6a, 6b, 6c and 6d show additional embodiments of the present invention. All figures are the front pelvic area of a female wearing the device of the present invention. Figures 6a, 6b, 6c and 6d show various covers which are generally attached over the front, stretchable elastic band 5 between the waistband 7 and the cover or protective cup 2 of the present invention. These various configurations of material generally have a hypo-allergenic inner side adjacent the body and a wax resistant outer side adapted to prevent wax applied during the waxing procedure from adhering to the surface. Any shape can be used to sculpt the pubic hair.
Figure 6a shows a lightning bolt configuration;
Figure 6b shows a heart-shaped configuration;
Figure 6c shows a triangular-shaped configuration; and
Figure 6d shows an arrow-shaped configuration.

The various figures shown in Figures 6a through 6d are used by the esthetician or operator carrying out the waxing procedure or laser hair removal, to sculpt the pubic hair into certain charming configurations.

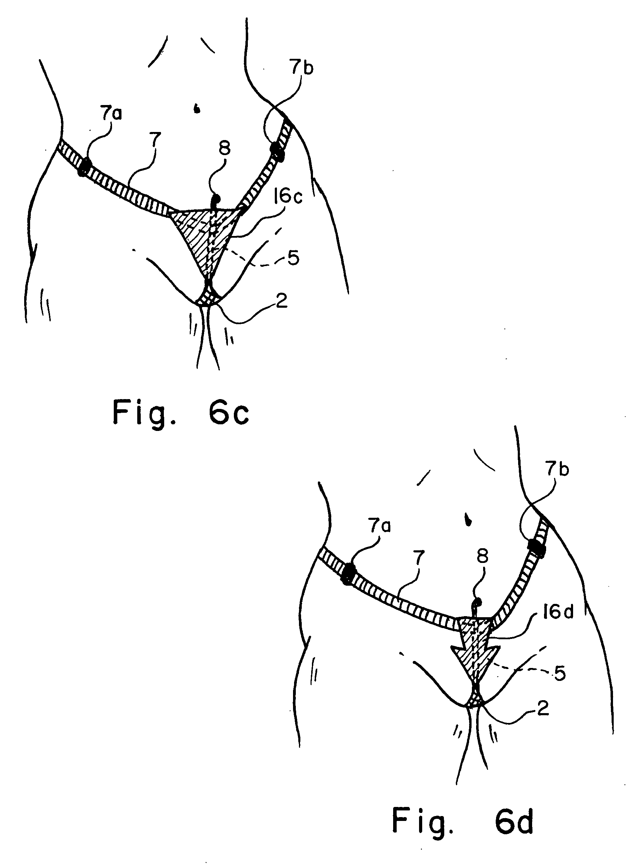

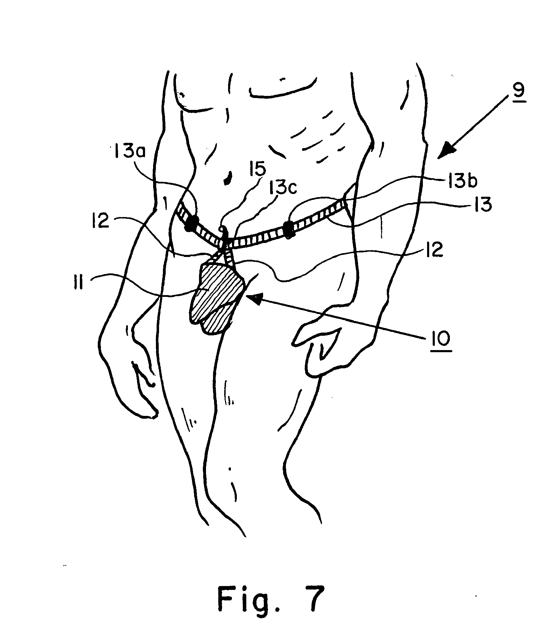

Figure 7 is a front perspective view of a male human body 9 wearing the device of the present invention. The cover of the male genitalia, generally shown as 10, is in the form of a pouch or pocket 11. The pouch 11 is upwardly attached to the waistband 13 by means of a pair of elastic, stretchable straps 12. These straps are pulled upwardly through aperture 13c of the waistband 13 to secure the strap by a knot 15. The waistband 13 in turn is adjustable by means of a pair of buckles 13a and 13b.

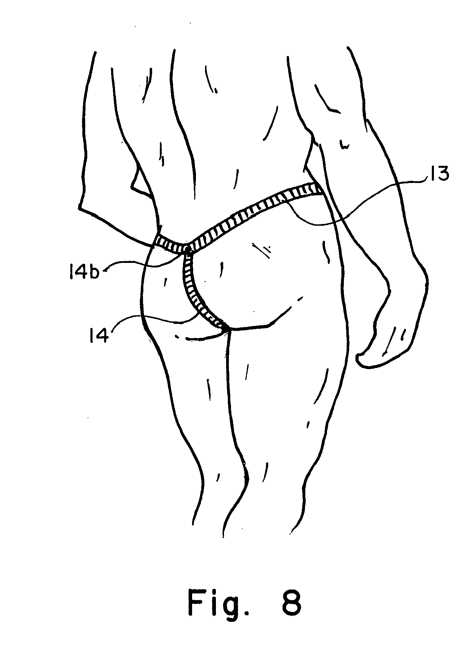

Figure 8 is a rear view of the male body shown in Figure 7 which indicates that a rear wide fluid impermeable strap 14 is attached to the waistband 13 by means of a clasp, Velcro or other means, at 14b.

Thus there has been shown, at least in the illustrated embodiments, a device for preventing transmission of human body fluids emitted from male and female genitalia and rectum during pubic hair-removal, comprising: a fluid impermeable cover; said cover adapted in operation to fit securely over at least inner labia of a female vulva or completely over a male penis; said device further comprising a fluid impermeable band secured to a lower end of said cover and adapted, in operation, to cover the rectum of a human; said cover and said fluid impermeable band being secured in place by a plurality of adjustable, stretchable straps.

## Claims

1. A device for preventing transmission of human body fluids emitted from male and female genitalia and rectum during pubic hair-removal, comprising: a fluid impermeable cover; said cover adapted in operation to fit securely over at least inner labia of a female vulva or completely over a male penis; said device further comprising a fluid impermeable band secured to a lower end of said cover and adapted, in operation, to cover the rectum of a human; said cover and said fluid impermeable band being secured in place by a plurality of adjustable, stretchable straps; said straps including at least one adjustable waistband, at least one adjustable, stretchable front strap, and optionally an adjustable stretchable rear strap; said front strap being attached at its lower end to an upper end of said cover and at its upper end stretchably secured to said waistband; said optional rear strap being attached at its lower end to an upper end of said fluid impermeable strap and at its upper end to said adjustable waistband.

2. A device as claimed in Claim 1 wherein said cover fits securely over the entire female vulva including both inner labia and outer labia.

3. A device as claimed in any of Claims 1 or 2 wherein said cover includes a hypo-allergenic inner lining, and a non-stick outer surface, wherein in operation, wax or ultrasound gel applied to surrounding body hair does not adhere to said outer surface.

4. A device as claimed in any of Claims 1, 2 or 3 wherein said wide, fluid impermeable band includes an inner hypo-allergenic material and an outer, non-stick surface; wherein, in operation, wax applied to surrounding body hair does not adhere to said outer surface.

5. A device as claimed in any preceding claim including at least two waistbands.

6. A device as claimed in any preceding claim wherein said cover for at least inner labia of a female vulva is an oval-shaped inverted cup.

7. A device as claimed in any of Claims 1 to 5, wherein said cover for male genitalia is configured as a pouch/pocket.

8. A device as claimed in any preceding claim wherein said waistband includes a central, front middle slit adapted to receive said stretchable front strap and in operation said strap is knotably secured to said waistband.

9. A device as claimed in any preceding claim wherein said waistband includes a central rear middle slit adapted to receive said adjustable, stretchable rear strap, and in operation, said strap is secured to said waistband.

10. A device as claimed in any preceding claim wherein at least one of said waistbands, said stretchable front strap, and said stretchable rear strap, includes adjustment tightening buckles.

11. A device as claimed in any of Claims 1 to 7 including two stretchable front straps.

12. A device as claimed in Claim 6 wherein a decorative shaped cover of any shape is integrated between said oval-shaped inverted cup and said waistband, wherein in operation said decorative shaped cover patterns removal of public hair.

13. A device as claimed in Claim 12 wherein said decorative shaped cover is one of a group of heart shaped, arrow shaped, triangular shaped, or lightning bolt shaped configurations.

14. A device as claimed in Claim 12 wherein said decorative shaped cover includes an inner hypoallergenic lining material and an outer non stick material which in operation, will not adhere to wax applied.

15. A cover as claimed in any preceding claim wherein a scented hypoallergenic strip is added to one side of said cover.

16. A device as claimed in any preceding claim wherein said cover and said wide fluid impermeable band are disposable; and said plurality of adjustable, stretchable, straps are detachably connected to said cover, and are reusable.
